# EUROPÄISCHE PATENTANMELDUNG

(11) **EP 0 908 185 A1**
(43) Veröffentlichungstag der Anmeldung: **14.04.1999**
(21) Anmeldenummer: 97117669.8
(22) Anmeldetag: 13.10.1997
(51) Int. Cl.: A61K 35/78

(54) **Verfahren zur Herstellung von Heilpflanzenextrakten**

(71) Anmelder: Max Zeller Söhne AG, 8590 Romanshorn (CH)
(72) Erfinder: Steiner, Rudolf, Prof. Dr.-Ing., 91056 Erlangen-Kosbach (DE); Hauk, Alexander, Dipl.-Ing., 92242 Hirschau (DE); Tratz, Werner, Dipl.-Ing., 91052 Erlangen (DE)
(74) Vertreter: Ritscher, Thomas, Dr.Rer.Nat.Dipl.-Chem.

(57) **Zusammenfassung**

Zur Herstellung von phytochemisch wirksamen Extrakten aus Pflanzenmaterial, insbesondere Petasites hybridus, das neben lipophilen Wirkstoffen auch Anteile an polaren oder potentiell polaren Komponenten, z.B. Akaloid-Anteile, wie Pyrrolizidine, enthält, durch Extraktion wird als Lösungsmittel flüssiges Kohlendioxid bei unterkritischen Bedingungen verwendet, da dieses die lipophilen Wirkstoffe besser löst, als die polaren Komponenten. Dadurch kann der bisher erforderliche Aufwand für die Reinigung des Extraktes signifikant vermindert werden.

Zur Durchführung des Verfahrens wird vorzugsweise mindestens ein druckfester Extraktionsbehälter (10) verwendet, in den flüssiges Kohlendioxid bei unterkritischen Bedingungen eingeführt und von dem ein Strom von Extrakt enthaltenden flüssigen Kohlendioxid abgezogen werden kann; ferner ist mindestens eine Zone (160;180) zur Behandlung des flüssigen Kohlendioxids mit Adsorptionsmittel vorgesehen, die im Extraktionsbehälter liegt oder/und diesen in einem separaten druckfesten Behälter (16) nachgeschaltet ist.

## Beschreibung

Die vorliegende Erfindung betrifft ein Verfahren zur Herstellung von phytochemisch wirksamen Extrakten aus einem Pflanzenmaterial, das neben lipophilen Wirkstoffen auch Anteile an polaren oder potentiell polaren Komponenten enthält, durch Extraktion mit einem selektiven Lösungsmittel, das die lipophilen Anteile besser löst, als die polaren Komponenten.

Die systematisch-wissenschaftliche Untersuchung der medizinischen Wirkung von Heilpflanzen ist allgemein Gegenstand der Phytotherapie, deren spezielle Aufgabe insbesondere die Verwendbarkeit und Gewinnung von aus Pflanzen gewonnen Präparaten zur therapeutischen Anwendung als Arzneimittel in der Human- und auch der Veterinärmedizin ist.

Trotz aller Fortschritte der Therapie mit halb- oder vollsynthetisch hergestellten Medikamenten hat die Phytotherapie einen festen Platz in der Behandlung von Krankheiten. Hierzu haben neuerdings verfügbare Methoden zur kritischen Beurteilung der therapeutischen Wirkungen von aus Pflanzen gewonnen Mitteln beigetragen, die es gestatten, eine über reine Placebo-Effekte hinausgehende Wirkung pharmakologisch zu sichern und zu bewerten. Ferner ist durch moderne analytische Methoden auch eine Standardisierung von direkt aus Pflanzen gewonnenen Produkten möglich, was in Anbetracht der insbesondere klimatisch bedingten Unterschiede von Wirkstoffart und Wirkstoffgehalt in Pflanzen aus verschiedenen Ernten für eine rationale Anwendung pflanzlicher Extrakte wesentlich ist.

Die hier verwendete Bezeichnung "phytochemisch wirksamer Extrakt" bezieht sich auf durch Extraktion aus Pflanzen gewonnene Produkte mit anerkannter therapeutischer Wirkung und definierter Qualität.

Hierbei werden als "Extrakte" sowohl primäre flüssige Extrakte als auch durch Lösungsmittelreduktion gewonnene "sekundäre" dickflüssige (spissum) sowie schliesslich die durch Lösungsmittelentfernung aus den flüssigen Extrakten erhältlichen festen Extrakte verstanden.

Bei der Gewinnung von Extrakten sind zwei Parameter von Bedeutung: die Extraktausbeute, d.h. die aus einer gegebenen Menge Pflanzenmaterial gewonne Menge an Extrakt, und die Extraktqualität, d.h. der Extrakt enthält die gewünschten (Wert) Stoffe und keine unerwünschten Komponenten.

Die Extraktqualität spielt insbesondere dann eine kritische Rolle, wenn das extrahierte Pflanzenmaterial Komponenten enthält, die störend oder gar toxisch sind, wie dies z.B. bei Petasites hybridus (Pestwurz) der Fall ist.

Therapeutische Verwendungen und Probleme der Gewinnung von Petasites-Extrakten sind in der Schweizer Patentanmeldung 0812/96 der Anmelderin ausführlich erläutert, auf welche Anmeldung hier durch Verweisung Bezug genommen wird. Darin sind insbesondere auch die aus DE-A-39 10 831 bzw. EP 0 391 504 und DE-A-41 41 749 bekannten Aspekte der kritischen Abtrennung der gewünschten Inhaltsstoffe ("Wertstoffe") von unerwünschten weil toxischen Pyrrolizidin-Alkaloiden ("Schadstoffe")einschliesslich der N-Oxide hiervon (nachfolgend gemeinsam kurz als PA bezeichnet) beschrieben.

Die Verwendung von selektiv wirkenden Extraktionsmitteln ist eine an sich übliche Methode für eine solche Trennung. Auf mehreren Gebieten der Verarbeitung und Behandlung von Pflanzenmaterial wird Kohlendioxid unter erhöhtem Druck als Extraktionsmittel verwendet, und es gibt eine umfangreiche Literatur über die selektive Extraktion von Tabak zur Gewinnung von Tabakaromastoffen (siehe z.B. DE-C2-31 48 335 und darin erwähnter Stand der Technik) sowie die Decoffeinierung von Kaffee und Tee (siehe z.B. Monographie von Stahl, E. et al., Verdichtete Gase zur Extraktion und Raffination, Springer Verlag 1987) mit Hilfe von Kohlendioxid als selektivem Extraktionsmittel.

In der Monographie von Stahl et al. wird dabei ausgeführt (Seite 199), dass die Extraktion mit flüssigem (d.h. unterkritischem) Kohlendioxid wegen der im Vergleich zu überkritischem Kohlendioxid geringeren Selektivität nicht zweckmässig ist. Tatsächlich wird dann auch in Übereinstimmung mit der Monographie von Stahl et al. in dem aus EP 0 391 504 bekannten Verfahren zur Gewinnung von Extrakten aus Petasites hybridus Kohlendioxid in überkritischem Zustand als selektives Lösungsmittel verwendet, um den PA-Anteil des Extraktes auf unter 0.1 ppm zu senken, was laut Patentschrift der Nachweisgrenze entsprechen soll.

Bei der Nacharbeitung der Angaben in der eben genannten Patentschrift konnten trotz verbesserter Analytik, die noch PA-Anteile von bis etwa 50 ppb (= 0.05 ppm) zu erfassen gestattet, unter den angegebenen Bedingungen solche niedrigen PA-Werte im unmittelbar gewonnen Extrakt nicht erzielt werden.

Im Zuge dieser Untersuchungen (Diplomarbeit Alexander Hauk, Herstellung von Pestwurzextrakten durch Extraktion mit hochverdichtetem CO₂, Lehrstuhl für Technische Chemie II, Friedrich-Alexander-Universität, Erlangen-Nürnberg) wurde jedoch gefunden, dass sich PA-Anteile des Extraktes von deutlich unter 0.1 ppm überraschenderweise dann erzielen lassen, wenn entgegen den Lehren des Standes der Technik Kohlendioxid gemäss der vorliegenden Erfindung in unterkritischem flüssigen Zustand als selektives Extraktionsmittel verwendet wird.

Gegenstand der Erfindung ist daher ein Verfahren zur Herstellung von phytochemisch wirksamen Extrakten aus Pflanzenmaterial, das wie Petasites hybridus neben lipophilen Wirkstoffen auch Anteile an polaren oder potentiell polaren Komponenten enthält, durch Extraktion mit einem selektiven Lösungsmittel, das die lipophilen Anteile besser löst, als die polaren Komponenten, und das dadurch gekennzeichnet ist, dass als Lösungsmittel Kohlendioxid bei unterkritischen Bedingungen verwendet wird.

Dadurch lässt sich der Aufwand zur Entfernung der PA-Anteile aus dem Extrakt signifikant verringern und die Wirtschaftlichkeit der Herstellung entsprechend verbessern.

Als polare oder potentiell polare Komponenten werden hier solche Anteile des Pflanzenmaterials verstanden, die ein signifikantes Dipolmoment haben, z.B. Komponenten mit basischen Gruppen, wie Alkaloide, die dem Molekül ein elektrisches Dipolmoment verleihen oder durch geeignete Behandlung stärker polar gemacht werden können. Folge der gegebenenfalls verstärkten Polarität ist ein entsprechend hydrophiler Charakter und dadurch eine bis zur Bedeutungslosigkeit verminderte Löslichkeit in flüssigem unterkritischem CO₂. Mit anderen Worten kann die Selektivität von flüssigem unterkritischem CO₂ für die Extraktion von lipophilen Komponenten um so mehr erhöht werden, als der polare Charakter der nicht im Extrakt gewünschten Komponenten verstärkt wird, und zwar überraschenderweise offenbar in höherem Masse, als dies bei überkritischem CO₂ der Fall ist.

Das besonders bevorzugte Pflanzenmaterial zur Verarbeitung nach dem erfindungsgemässen Verfahren ist ein solches von Petasites sp., insbesondere Petasites hybridus, das bekanntlich Anteile an Pyrrolizidin-Akaloidverbindungen (PA) enthält.

Allgemein wird dass flüssiges Kohlendioxid als Extraktionsmittel beim erfindungsgemässen Verfahren unter einem Druck von < 73.83 bar, vorzugsweise ≤ 73.5 bar, insbesondere 1 - 70 bar, und einer Temperatur von < 31.06 °C, vorzugsweise ≤ 31 °C, insbesondere 0 - 30°C, verwendet.

Gemäss einer allgemein bevorzugten Ausführungsform des erfindungsgemässen Verfahrens wird das extrahierte Pflanzenmaterial nach der Behandlung mit dem flüssigen Kohlendioxid von diesem getrennt und letzteres zur Gewinnung eines im wesentlichen von Wasser (einer ebenfalls polaren Komponente) und Alkaloiden freien Extraktes abgedampft, z.B. in einem Expansionsraum bei Temperaturen von über 31°C.

Bei der Durchführung des erfindungsgemässen Verfahrens kann es zur weiteren Verringerung des Anteils an unerwünschten Alkaloidverbindungen im Extrakt zweckmässig sein, das vom extrahierten Pflanzenmaterial abgetrennte aber Extrakt enthaltende flüssige Kohlendioxid mit einem Adsorptionsmittel zu behandeln, um allfällige restliche polare Anteile aus dem flüssigen Kohlendioxid zu entfernen. Dies kann durch Rezyklieren während der Adsorption oder nachfolgend geschehen.

Als Adsorptionsmittel sind neben den typischen Adsorbentien auch Ionenaustauscher zur Entfernung von Pyrrolizidin-Alkaloiden und/oder deren N-Oxiden geeignet, vorzugsweise Kationenaustauscher in H⁺-Form, z.B. ein solcher auf Basis eines sauren Polystyrolharzes, wie es technisch unter der Bezeichnung Lewatit® SC 104 (Bayer-Katalysator K 1221 der Firma Bayer AG erhältlich ist, oder ein makroretikularer Ionenaustauscher, wie er z.B. unter der Bezeichnung Amberlyst® von der Firma Rohm & Haas erhältlich ist. Die Wahl und Verwendung von Adsorptionsmitteln für ein gegebenes Material liegt im Rahmen des fachlichen Könnens und kann durch einige einfache Versuche optimiert werden.

Das Pflanzenmaterial wird vor der erfindungsgemässen Extraktion meist mechanisch, z.B. durch Zerkleinerung in einer Mahlanlage, z.B. einer Mühle geeigneter Bauart oder/und einem Walzenstuhl, vorbehandelt, kann aber auch chemisch oder mechanisch/chemisch vorbehandelt werden, z.B. durch Behandlung mit Säuren, Basen oder Enzymen, mit oder ohne gleichzeitige mechanische Einwirkungen.

Gegenstand der Erfindung ist ferner ein phytotherapeutisches Mittel, das mindestens teilweise aus Extrakt von Petasites hybridus besteht oder daraus gewonnen ist, welcher Extrakt nach dem erfindungsgemässen Verfahren erhalten ist, insbesondere ein Mittel, dessen Pyrrolizidin-Anteil im Extrakt (einschliesslich der N-Oxide) unter etwa 75 ppb (0.075 ppm) und typisch im Bereich von etwa 50 ppb oder weniger liegt.

Gegenstand der Erfindung ist ferner die Anwendung des erfindungsgemässen Verfahrens zur Extraktion von Pflanzenmaterial, das wie Petasites hybridus phytochemisch wirksame lipophile Anteile und unerwünschte polare oder potentiell polare Komponenten enthält.

Die chemische Zusammensetzung und die Strukturen der Komponenten der bevorzugten Extrakte aus Petasites hybridus sind für die Petasin-Varietät insbesondere aus Chimia 48 (1994) S. 564 - 569 bekannt (siehe dort insbesondere die Formeln 1 - 4 und Tabellen 2 und 3), auf die hier durch Verweisung Bezug genommen wird. Neben Petasin finden sich in den Extrakten meist eine Reihe petasinähnlicher Stoffe, wie Isopetasin, Neopetasin, Desoxyneopetasol, Desoxyiso- und neopetasol (einschliesslich der 13-substituierten Derivate hiervon), Methylcrotonylpetasol und -isopetasol, Methacryloyl-petasol und -isopetasol, Isobutyryl-neopetasol sowie Methylthioacryloyl-petasol, -neopetasol und -isopetasol.

Für die Furanopetasin-Varietät von Petasites hybridus sind die Strukturen der wichtigsten Komponenten (frisch bzw. gefriergetrocknet und luftgetrocknet) aus einer Dissertation (Siegenthaler; Untersuchungen zur Struktur und Analytik der Inhaltsstoffe von Petasites albus und Petasites hybridus (Furanopetasin-Varietät); Bern 1995 )bekannt. Es handelt sich dabei insbesondere um die Verbindungsgruppen der Furanoeremophilane und Furanoeremophilanlactone (s. Tabellen auf Seiten 198 und 199 der Dissertation Siegenthaler).

Zur Standardisierung erfindungsgemäss verwendeter Extrakte kann zweckmässig auf den Petasin/Isopetasingehalt (einschliesslich der entsprechenden Derivate, wie S-Petasin) bzw. auf den Furanoeremophilan-Gehalt (einschliesslich der entsprechenden Derivate) Bezug genommen werden.

Zur Durchführung des erfindungsgemässen Verfahrens sind grundsätzlich diejenigen Vorrichtungen und Anlagen geeignet, die auch für die Extraktion mit überkritischem Kohlendioxid verwendet werden können; allgemein erforderlich ist, dass die Anlagen den speziell gewählten unterkritischen Bedingungen entsprechen müssen, was aber eine für Fachleute ohne weiteres erfüllbare Anweisung darstellt.

Eine zur Durchführung des neuen Verfahrens bevorzugte erfindungsgemässe Vorrichtung besitzt mindestens einen druckfesten Extraktionsbehälter, in den flüssiges Kohlendioxid bei unterkritischen Bedingungen eingeführt und von dem ein Strom von Extrakt enthaltendem flüssigen Kohlendioxid abgezogen werden kann, und ist gekennzeichnet durch mindestens eine Zone zur Behandlung des extrakthaltigen flüssigen Kohlendioxids mit Adsorptionsmittel, wobei die Zone im Extraktionsbehälter liegt oder/und diesem in einem separaten druckfesten Behälter nachgeschaltet ist.

Die Erfindung wird nachfolgend anhand der Zeichnung und anhand von Beispielen erläutert, ohne dass dies eine Beschränkung darstellt.

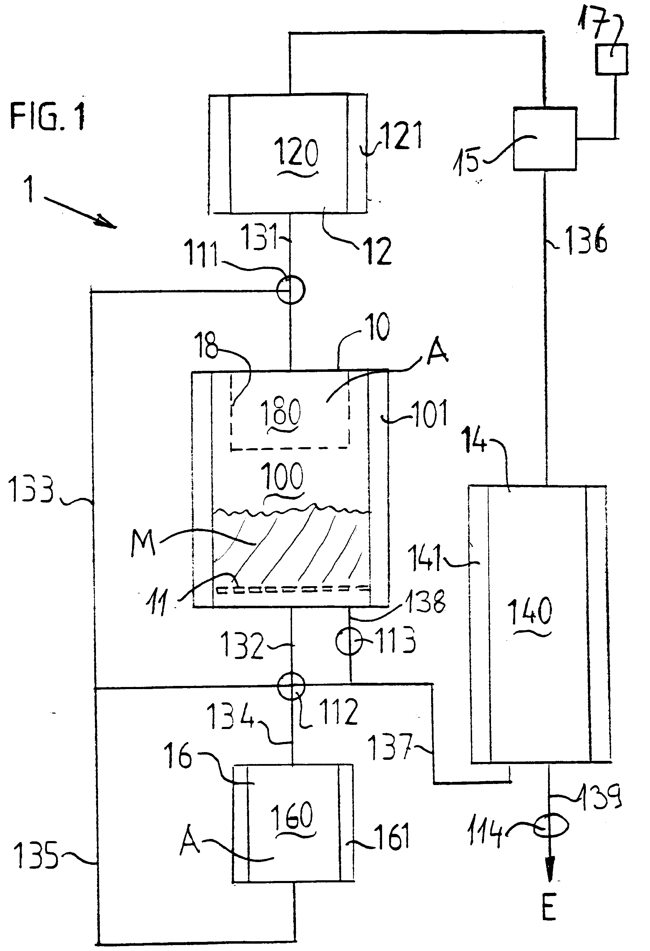

In der einzigen Figur 1 ist das Schema einer Vorrichtung 1 zur Durchführung des Verfahrens dargestellt. Die Vorrichtung 1 umfasst mindestens einen Extraktionsbehälter 10 (nachfolgend kurz Extraktor genannt), der ausreichend druckbeständig zum Betrieb mit flüssigem Kohlendioxid bei unterkritischen Bedingungen geeignet ist. Allgemein sollten der Extraktor und alle den Extrakt führenden und aufnehmenden Teile einer zur Durchführung des erfindungsgemässen Verfahrens verwendeten Anlage einen Betriebsdruck von mindestens 50 bar und vorzugsweise etwa 100 bar zulassen.

Der Extraktor 10 hat einen Innenraum 100 zur Aufnahme und Rückhaltung von zerkleinertem Pflanzenmaterial M und einen Doppelmantel 101 zur Temperierung des Innenraums 100. Ferner ist der Extraktor 10 mit Mess- und Regeleinrichtungen versehen, um die Einhaltung einer gegebenen Extraktionstemperatur bei unterkritischen Bedingungen des flüssigen CO₂ zu gewährleisten. Im Extraktor 10 ist ein für flüssiges Kohlendioxid durchlässiger Boden 11 angebracht, der das in den Extraktionsraum 100 eingeführte Pflanzenmaterial im Extraktor 10 zurückhält.

Der Extraktor 10 ist durch die Leitung 131 über das Ventil 111 mit einer Quelle 12 für flüssiges Kohlendioxid verbunden. Die Quelle 12 ist im typischen Fall ein druckfester (z.B. bis 150 bar) Behälter, der mit einem Doppelmantel 121 zur Temperierung und mit (nicht dargestellten) Temperatur- und Druckmesseinrichtungen versehen ist. Die Quelle 12 ist ferner mit einer Anlage 15 zur Verflüssigung von gasförmigem Kohlendioxid verbunden, die über die Leitung 136 mit rezykliertem gasförmigem CO₂ gespeist wird. Zur Erstspeisung mit bzw. Ergänzung von CO₂ kann die Anlage 15 mit einem Druckbehälter 17 verbunden werden, der gasförmiges CO₂ enthält.

In der Leitung 131 ist ein Umschaltventil 111 angeordnet, um die Zufuhr von Kohlendioxid von der Quelle 12 zu drosseln oder abzuschalten, wenn der den Extraktor 10 durch die Leitung 132 verlassende Strom aus flüssigem Kohlendioxid mit darin enthaltenen Extraktmaterial (Extraktionsstrom) über das Steuerventil 112 und die Leitung 133 in den Extraktor 10 rezirkuliert wird.

Wenn der Extraktionsstrom gemäss einer bevorzugten Ausführungsform des erfindungsgemässen Verfahrens mit festem Adsorptionsmittel A behandelt werden soll, kann dies in einem separaten Behälter 16 geschehen, der das Absorptionsmittel in der Zone 160 enthält und durch die Leitung 134 mit dem Extraktionsstrom gespeist wird, indem das Ventil 112 in die zur Beschickung des Behälters 16 durch die Leitung 134 mit dem gesamten Extraktionsstrom oder einem Teil desselben erforderliche Stellung gebracht worden ist. Der den Behälter 16 verlassende Extraktionsstrom gelangt dann durch die Leitung 135 in die Leitung 133 und wieder in den Extraktor 10.

Alternativ oder komplementär kann Adsorptionsmittel A auch in einer Zone 180 vorgelegt werden, die im Extraktor 10 liegt und z.B. von einem Einsatz 18 gebildet wird, der im oberen Teil des Extraktors 10 liegt und für das flüssige Kohlendioxid durchlässig ist, das Adsorptionsmittel aber zurückhält.

Bein Erreichen der gewünschten Extraktkonzentration kann der Extraktionsstrom entweder durch die Leitung 138 und das Ventil 113 oder die Leitung 132 und das entsprechend umgestellte Ventil 112 in die Leitung 137 und in den Expansionsbehälter 14 überführt werden, in dessen Innenraum 140 das CO₂ verdampft wird und dadurch der über die Leitung 139 und das Ventil 114 abziehbare Extrakt E gewonnen wird. Der Expansionsbehälter 14 ist mit einem Heizmantel 141 versehen, um die zur Verdampfung des CO₂ erforderliche Wärme zuzuführen. Das gasförmige CO₂ gelangt dann über die Leitung 136 in die Verflüssigungsanlage 15 und von dort in den Vorratsbehälter 12.

Die unerwünschten polaren Anteile, d.h. in vorliegenden Fall hauptsächlich Pyrrolizidin-Alkaloide und Wasser, verbleiben im Extraktionsrückstand, der in üblicher Weise durch Verbrennen oder durch Verwendung als Dünger entsorgt werden kann.

Die Einzelheiten der Steuerung und Überwachung Extraktionsvorgangs in der Anlage 1 und der Energiekonservierung beim Verdampfen und Verflüssigen des Kohlendioxids durch entsprechende Wärmetauscher sind in Fig. 1 nicht dargestellt, da sie im Bereich des Fachwissens liegen.

Die nachfolgenden Ausführungen dienen der weiteren Erläuterung der Erfindung, ohne diese zu beschränken.

### BEISPIELE

Die Beispiele wurden in einer Anlage durchgeführt, die im Prinzip dem in Figur 1 dargestellten Schema entsprach. Die der Quelle 12 für flüssiges Kohlendioxid vorgeschaltete Verflüssigungsanlage 15 wurde mit gasförmigen handelsüblichem Kohlendioxid, z.B. aus üblichen Bomben 17, gespeist. Die Quelle selbst war für einen maximalen Betriebsdruck von etwa 150 bar ausgelegt und mit einem äusseren Kühlmantel 121 und einer (in Fig. 1 nicht dargestellten) inneren Kühlschlange versehen. Als Kühlmittel diente ein Glykol-Wassergemisch.

Der Extraktor 10 war ein Hochdruck-Autoklav der Firma Nowa Swiss (Effretikon, Schweiz) aus rostfreiem Stahl mit einem zylindrischen Innenraum und einem Volumen von 400 ml. Der CO₂-Strom wurde mit einem Durchflussmessgerät (Nicro-Motion) bestimmt. Als Förderpumpen wurden Membranpumpen (Nominalleistung 10.9 Liter/Stunde) verwendet. Einstellung und Regelung des Arbeitsdrucks im Extraktor 10 erfolgte über ein Dosierventil mit Schrittmotor. Für die Druckmessung wurde ein Präzisionsmanometer (Manom AG, Hitzkirch) verwendet. Der Betrieb der Anlage konnte manuell oder automatisch gesteuert werden.

Das für die Versuche als Ausgangsmaterial verwendete Pflanzenmaterial bestand aus Wurzelstock (Rhizom) von Petasites hybridus, der bis zu einer mittleren Korngrösse von etwa 500µm zerkleinert worden war.

Als Charge wurden 160 g Pflanzenmaterial (Feuchtigkeitsgehalt 6 bzw. 9 Gew.%) in den Extraktor eingefüllt und 30 Minuten mit flüssigem Kohlendioxid bei 65 bar und 20°C mit einem CO₂-Durchsatz von 25 kg CO₂/kg Pflanzenmaterial·Stunde extrahiert. Der Schadstoffgehalt des Pflanzenmaterials (Pyrrolizidin-Alkaloide und N-Oxide) betrug 40 ppm (40'000 ppb).

### Beispiel 1

Bei diesem Versuch wurde das Ausgangsmaterial als solches verwendet, d.h. weder vorbehandelt noch der Extrakt mit einen Adsorbens nachgereinigt. Es wurden 5,03 g eines klaren hellgelben, viskosen Petasites-Extraktes mit einem Gehalt an Pyrrolizidin-Alkaloiden von 0.874 ppm (=874 ppb) erhalten.

### Beispiel 2

Bei diesem Versuch wurde das Ausgangsmaterial in einem Vorbehandlungsschritt mit 6 g 0,1 N-H₂SO₄ besprüht und etwa 15 Stunden zur Einwirkung der Säure stehengelassen. Dann wurde es in den Autoklaven eingefüllt und wie beschrieben mit flüssigen CO₂ extrahiert. Es wurden 4,64 g eines klaren, hellgelben, viskosen Petasites-Extraktes mit einem PA-Gehalt von 566 ppb erhalten.

### Beispiel 3

Bei diesen Versuch wurde das Ausgangsmaterial nicht vorbehandelt, aber es wurden zusätzlich zu den 160 g zerkleinerten Pestwurz-Rhizomen noch 70 g des stark sauren Kationentauscherharzes Bayer Katalysatorkomponente K 1221 (früher Lewatit SC 104) in den Adsorbensbehälter 18 geschichtet. Bei der Extraktion wurden 5,00 g eines ebenfalls klaren, hellgelben, viskosen Petasites-Extrakts mit einem PA-Gehalt von 133 ppb erhalten.

### Beispiel 4

Bein diesem Versuch wurde das Ausgangsmaterial wie in Beispiel 2 mit 6 g 0,1 N-H₂SO₄ besprüht und anschliessend etwa 15 h zur Einwirkung der Säure stehengelassen. Wie in Beispiel 3 wurde die Adsorbens-Zone 180 des Extraktors 10 mit 70 g Adsorberharz beschickt; dann wurde mit flüssigem CO₂ extrahiert. Es wurden 4,53 g Patasites-Extrakt mit einem PA-Gehalt von 37 ppb (= 0.037 ppm) erhalten. Dieser Wert liegt im Bereich der Nachweisgrenze der angewendeten Analysenmethodik (GC-Analyse).

Allgemein werden die qualitativ hochwertigsten Extrakte mit PA-Gehalten unter 0.075 ppm (75 ppb) erhalten, wenn die zerkleinerten Pestwurz-Rhizome durch Säureeinwirkung vorbehandelt, mit unterkritischem flüssigem CO₂ extrahiert und der rezirkulierte Extraktstrom mit Adsorbens behandelt wird.

### VERGLEICHSBEISPIELE

Um die gegenüber den Stand der Technik verbesserte Wirkungsweise der erfindungsgemässen Extraktion mit flüssigem unterkritischem CO₂ zu belegen, wurden Vergleichsversuche unter grundsätzlich gleichen Bedingungen wie in den obigen erfindungsgemässen Beispielen durchgeführt. Unterschiedlich waren lediglich die Druck-Temperatur-Bedingungen, die mit 250 bar und 45°C über dem kritischen Punkt des CO₂ (74 bar /31°C) lagen und damit dem Stand der Technik entsprachen.

### Vergleichsbeispiel 5 (Vergleich mit Beispiel 1)

Bei diesem Versuch wurde das Ausgangsmaterial wie in Beispiel 1 nicht vorbehandelt und auch nicht adsorptiv nachgereinigt. Aus 160 g zerkleinerten Pestwurz-Rhizomen wurden 6,64 g eines milchig-gelben Petasites-Extraktes mit einem Wassergehalt von ca. 20% und einem PA-Gehalt von 5.680 ppm (= 5680 ppb) gewonnen.

Dieser Vergleich zeigt, dass die erfindungsgemässe Extraktion mit unterkritischem flüssigem CO₂ bereits ohne Vor- oder Nachbehandlung entscheidende Vorteile gegenüber dem Stand der Technik hat: zwar ist die Extraktausbeute bei der Extraktion mit überkritischen CO₂ höher, aber der dabei erhaltene Extrakt enthält erhebliche Mengen Wasser, ist deshalb milchig gefärbt und müsste nachträglich in eine wässrige und eine organische Phase getrennt werden, was einen zusätzlichen Aufwand erfordert.

Selbst nach Abtrennung der wässrigen Phase enthält die Wertstoffextraktion noch 3920 ppb PA. Bei der Extraktion mit flüssigem unterkritischem CO₂ wird hingegen nur eine organische Phase erhalten, die praktisch wasserfrei ist, weil das flüssige CO₂ das in Ausgangsmaterial enthaltene Wasser, das ein polarer Anteil des Pflanzenmaterials ist, nicht mitextrahiert. Der erfindungsgemäss erhaltene Extrakt enthält mit nur 874 ppb PA einen signifikant niedrigeren Schadstoffgehalt als das Produkt aus der Extraktion mit überkritischem CO₂.

### Vergleichsbeispiel 6 (Vergleich mit Beispiel 3)

Bei diesem Versuch wurde das Ausgangsmaterial wie in Beispiel 3 nicht vorbehandelt, der Adsorbensraum 180 aber ebenfalls mit 70 g Adsorberharz gefüllt. Es wurden 5,36 g eines klaren, aber dunkelgelben Petasites-Extrakts mit einem PA-Gehalt von 0.9 ppm (= 900 ppb) erhalten.

Auch dieser Vergleich zeigt deutlich die Vorteile der erfindungsgemässen Extraktion gegenüber der bekannten Extraktion mit überkritischem flüssigem Kohlendioxid. Zwar werden in beiden Fällen klare , d.h. praktisch wasserfreie Extrakte erhalten. Dies ist bei Extraktion mit überkritischem CO₂ gemäss Stand der Technik vermutlich im wesentlichen dadurch bedingt, dass das Wasser durch das Adsorbens aufgenommen wird. Beim erfindungsgemässen Verfahren ist die Entfernung von Wasser hingegen dem polaren Charakter von Wasser und der diesbezüglich negativen Selektivität von flüssigem unterkritischem CO₂ zuzuschreiben. Der erfindungsgemäss erhaltene Extrakt ist aber ausserdem deutlich heller und enthält vor allen signifikant weniger PA, nämlich nur 133 ppb gegenüber 900 ppb bei Verwendung von überkritischen CO₂ unter sonst gleichen Bedingungen.

Die Ergebnisse der obigen Beispiele sind in der nachfolgenden Tabelle zusammengestellt.

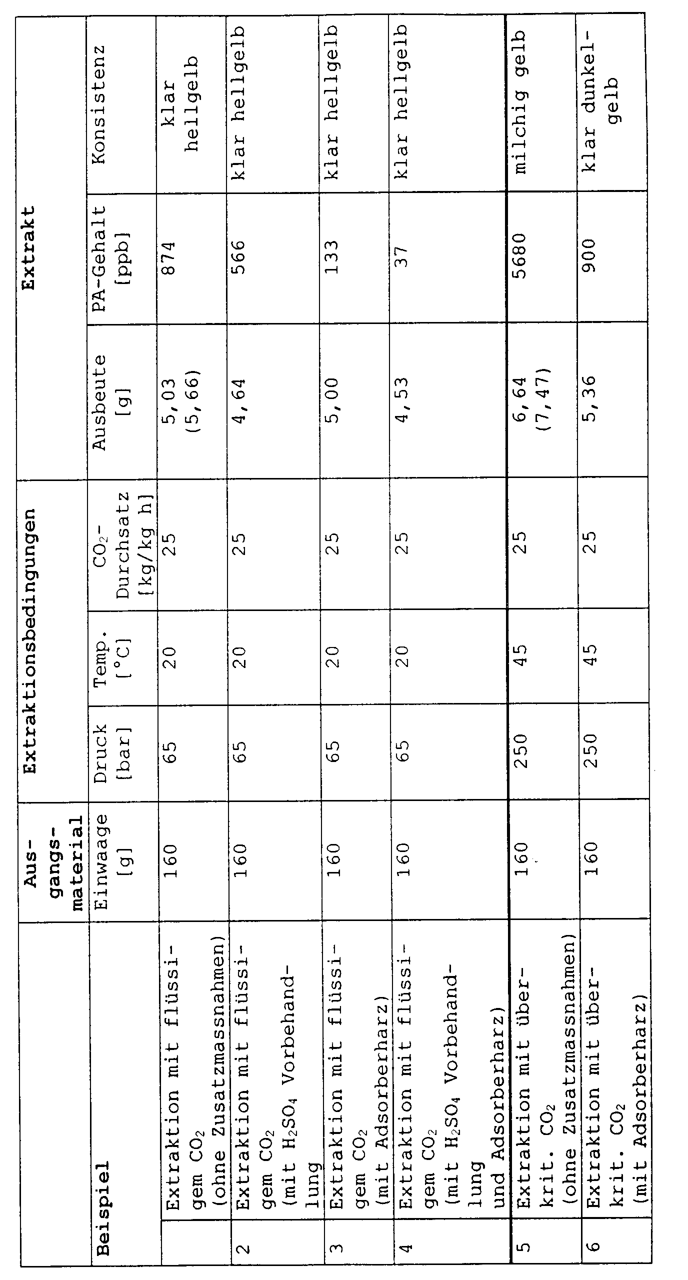

Für Fachleute ergeben sich aus der obigen Beschreibung verschiedene Variationen des beschriebenen neuen Verfahrens, die jedoch im Rahmen des Schutzumfangs der nachfolgenden Ansprüche liegen. So kann z.B. die oben beschriebene chargenweise Extraktion kontinuierlich durchgeführt werden, z.B. indem mehrere Extraktoren parallel geschaltet und je für sich chargenweise betrieben werden, aber insgesamt ein kontinuierlicher Produktstrom entsteht. Alternativ können Hochdruckextraktoren für kontinuierlichen Betrieb und vorzugsweise im Gegenstrom erfolgenden Betrieb verwendet werden, indem das am stärksten extrahierte Pflanzenmaterial mit unbeladenem flüssigen unterkritischen CO₂ und das frische Pflanzenmaterial mit dem bereits extrakthaltigen CO₂ behandelt wird. Anstelle von Petasites hybridus können andere bekannte Heilmittelpflanzen erfindungsgemäss extrahiert werden. Weitere Anpassungen an gegebene spezielle Verhältnisse sowie die Weiterverarbeitung der erhaltenen Extrakte liegen in Bereich des Fachmännischen und bedürfen keiner Erläuterung.

## Patentansprüche

1. Verfahren zur Herstellung von phytochemisch wirksamen Extrakten aus einen Pflanzenmaterial, das neben lipophilen Wirkstoffen auch Anteile an polaren oder potentiell polaren Komponenten enthält, durch Extraktion mit einem Lösungsmittel, das die lipophilen Anteile besser löst, als die polaren Komponenten, dadurch gekennzeichnet, dass als Lösungsmittel flüssiges Kohlendioxid bei unterkritischen Bedingungen verwendet wird.

2. Verfahren nach Anspruch 1, dadurch gekennzeichnet, dass das Pflanzenmaterial als polare Komponente(n) Wasser und/oder mindestens ein Alkaloid enthält, das polar ist oder durch sauren oder alkalischen Aufschluss polar gemacht werden kann.

3. Verfahren nach Anspruch 1 oder 2, dadurch gekennzeichnet, dass als Pflanzenmaterial ein solches von Petasites sp., insbesondere Petasites hybridus, verwendet wird, das Anteile an Pyrrolizidin-Verbindungen und/oder deren N-Oxide ethält.

4. Verfahren nach einem der Ansprüche 1 - 3, dadurch gekennzeichnet, dass flüssiges Kohlendioxid bei einem Druck von < 73.83 bar, vorzugsweise ≤ 73.5 bar, insbesondere 1 - 70 bar, und einer Temperatur von < 31.06 °C, vorzugsweise ≤ 31 °C, insbesondere 0 - 30°C, als Extraktionsmittel verwendet wird.

5. Verfahren nach einem der Ansprüche 1 - 4, dadurch gekennzeichnet, dass das Pflanzenmaterial nach der Behandlung mit dem flüssigen Kohlendioxid von diesem getrennt und letzteres zur Gewinnung eines im wesentlichen von Wasser und Alkaloiden freien Extraktes abgedampft wird, z.B. in einem Autoklav bei Temperaturen von über 31°C.

6. Verfahren nach Anspruch 5, dadurch gekennzeichnet, dass das flüssige Kohlendioxid mit darin gelösten Anteilen des Pflanzenmaterials während oder nach der Extraktion mit einen Adsorptionsmittel behandelt wird, um restliche Anteile polarer Komponenten des Extrakts zu entfernen.

7. Verfahren nach einem der Ansprüche 1 - 6, dadurch gekennzeichnet, dass das Pflanzenmaterial mechanisch, z.B. durch Zerkleinerung, und/oder chemisch, z.B. durch Behandlung mit Säuren, Basen, Enzymen oder Ionenaustauschern, vorbehandelt wird.

8. Phytotherapeutisches Mittel, das mindestens teilweise aus Extrakt von Petasites hybridus besteht oder daraus gewonnen ist, dadurch gekennzeichnet, dass der Extrakt nach den Verfahren gemäss einen der Ansprüche 1 - 7 gewonnen ist.

9. Mittel nach Anspruch 8, dadurch gekennzeichnet, dass der Pyrrolizidin-Anteil einschliesslich von N-Oxiden hiervon ≤ 0.075 ppm beträgt und vorzugsweise in Bereich von etwa 50 ppb oder darunter liegt.

10. Vorrichtung zur Durchführung des Verfahrens nach einen der Ansprüche 1 - 7 mit mindestens einem druckfesten Extraktionsbehälter, in den flüssiges Kohlendioxid bei unterkritischen Bedingungen eingeführt und von dem ein Strom von Extrakt enthaltenden flüssigen Kohlendioxid abgezogen werden kann, gekennzeichnet durch mindestens eine Zone zur Behandlung des extrakthaltigen flüssigen Kohlendioxids mit Adsorptionsmittel, wobei die Zone im Extraktionsbehälter liegt oder/und diesem in einem separaten druckfesten Behälter nachgeschaltet ist.
